Europäisches Patentamt

European Patent Office

Office européen des brevets

(11) Veröffentlichungsnummer: **0 225 535 B1**

# EUROPÄISCHE PATENTSCHRIFT

(45) Veröffentlichungstag der Patentschrift: **15.05.91**

(21) Anmeldenummer: **86116244.4**

(22) Anmeldetag: **24.11.86**

(51) Int. Cl.5: **C08F 220/32, C12N 11/08, B01J 41/14, C08F 216/14**

(54) **Perlförmige vernetzte, Epoxy- und basische Aminogruppen aufweisende Mischpolymerisate, Verfahren zu ihrer Herstellung und ihre Verwendung.**

(30) Priorität: **07.12.85 DE 3543348**

(43) Veröffentlichungstag der Anmeldung:
**16.06.87 Patentblatt 87/25**

(45) Bekanntmachung des Hinweises auf die
Patenterteilung:
**15.05.91 Patentblatt 91/20**

(84) Benannte Vertragsstaaten:
**CH DE ES FR GB IT LI SE**

(56) Entgegenhaltungen:
**EP-A- 0 058 767**

**SOVIET INVENTIONS ILLUSTRATED, Sektion Chemical, Januar 1967, Seite 17, Nr. 68-82804P(00), Derwent Publications LTD**

(73) Patentinhaber: **BAYER AG**

**W-5090 Leverkusen 1 Bayerwerk(DE)**

(72) Erfinder: **Mitschker, Alfred, Dr.**
**Am Gartenfeld 50**
**W-5068 Odenthal-Holz(DE)**
Erfinder: **Lange, Peter Michael, Dr.**
**Walter-Flex-Strasse 9**
**W-5090 Leverkusen(DE)**
Erfinder: **Kreiss, Wolfgang, Dr.**
**z.Zt. Mobay Corporation PU-Research State Route 2**
**New Martinsville, W.V. 26155(US)**

## Beschreibung

Die Erfindung betrifft neue makroporöse perlförmige vernetzte Epoxy- und basische Aminogruppen aufweisende Mischpolymerisate, ein Verfahren zu ihrer Herstellung und ihre Verwendung.

Perlpolymerisate vernetzter, Epoxygruppen aufweisender Mischpolymerisate sind bekannt. So sind z.B. in der DE-AS 22 37 316, den DE-OS 23 43 633 und 27 22 751 und der EP-B1-0 058 767 verschiedene, durch inverse Perlpolymerisation hergestellte, hydrophile, vernetzte, Epoxygruppen aufweisende Mischpolymerisate beschrieben. Diese Mischpolymerisate sind aus gegenüber Proteinen bindungsaktiven Monomeren, z.B. Glycidyl-(meth-)acrylaten, oder Glycidylvinyl-oder -allylethern, vernetzenden Monomeren wie Glykol-di-(meth-)acrylat und/oder Methylen-bis-(meth-)acrylamid und hydrophilen Monomeren, z.B. Hydroxygruppen aufweisenden Monomeren, aufgebaut. Gemäß DE-OS 27 22 751 wird durch die Anwendung bestimmter Mengen an vernetzenden Monomeren und (Meth-)Acrylamiden die Ausbildung von Hohlperlen erreicht. Gemäß EP-B1-0 058 767 werden durch Anwendung bestimmter Verdünnungsmittelgemische bei der inversen Perlpolymerisation Mischpolymerisate mit besonders hoher Bindungskapazität für bestimmte Enzyme erhalten.

Aus der DE-PS 27 28 146 sind gelartige, makroporöse Perlpolymerisate vernetzter, Epoxygruppen aufweisender Mischpolymerisate aus Glycidylmonovinylestern oder Glycidylmonovinylethern und Alkylenglykoldivinylestern bekannt. Diese Mischpolymerisate werden durch radikalische wäßrige Suspensionspolymerisation der Monomeren in Gegenwart die Monomere lösender organischer Verdünnungsmittel hergestellt und durch Hydrolyse oder Alkoholyse der Epoxygruppen in Epoxygruppen-freie modifizierte Mischpolymerisate überführt.

In der Angew. Makromol. Chemie 117 (1983) 117-129 werden makroporöse Perlpolymerisate vernetzter, Epoxygruppen aufweisender Mischpolymerisate aus Glycidylmethacrylat und Ethylendimethacrylat beschrieben. Sie werden durch übliche Perlpolymerisation in wäßriger Suspension in Gegenwart verschiedener Porösmacher hergestellt.

Es wurde nun gefunden, daß man zu neuen, vielseitig verwendbaren makroporösen, vernetzten, Epoxygruppen aufweisenden Mischpolymerisaten mit günstigen Bindungseigenschaften für biologisch aktive Substanzen gelangt, und dies durch übliche Perlpolymerisation in wäßriger Suspension, wenn man polymerisierbare Glycidylverbindungen wie Glycidyl-(meth-)acrylate, Glycidyl-vinyl- und/oder Glycidyl-allylether vor oder während der radikalischen Perlpolymerisation, die in Gegenwart von Porösmachern und, gegebenenfalls unter Mitverwendung von radikalisch polymerisierbaren Polyvinylverbindungen vorgenommen wird, mit Aminen umsetzt, die mit mindestens zwei Epoxygruppen unter Öffnung des Epoxyringes zu reagieren vermögen. Durch die Verwendung der Amine, die mit mindestens zwei Epoxygruppen zu reagieren vermögen, werden perlförmige Mischpolymerisate erhalten, die günstigere Bindungseigenschaften für biologisch aktive Substanzen aufweisen und vielseitiger anwendbar sind als die vorbekannten perlförmigen Mischpolymerisate auf Basis polymerisierbarer Glycidylverbindungen, die außer Epoxygruppen nur noch Hydroxygruppen aufweisen. Die Amine dienen gleichzeitig als Vernetzer und als basische Modifikatoren für die polymerisierbaren Glycidylverbindungen. Außerdem bewirken die Amine die Ausbildung besonderer Porenstrukturen im Mischpolymerisat.

Die Erfindung betrifft daher neue perlförmige makroporöse vernetzte Epoxy- und basische Aminogruppen aufweisende Mischpolymerisate, die dadurch erhältlich sind, daß man polymerisierbare Glycidylverbindungen, vorzugsweise Glycidyl-(meth-)acrylate, Glycidylvinylether und/oder Glycidylallylether, vor oder während der radikalischen Perlpolymerisation, die in Gegenwart von Porösmachern und gegebenenfalls unter Zusatz von radikalisch polymerisierbaren Polyvinylverbindungen vorgenommen wird, mit Aminen umsetzt, die mit mindestens zwei Epoxygruppen zu reagieren vermögen.

Die Erfindung betrifft ferner ein Verfahren zur Herstellung dieser neuen makroporösen, perlförmigen, vernetzten Epoxy- und basische Aminogruppen aufweisenden Mischpolymerisate; das Verfahren ist dadurch gekennzeichnet, daß man polymerisierbare Glycidylverbindungen, gegebenenfalls unter Zusatz von radikalisch polymerisierbaren Polyvinylverbindungen, in wäßriger Suspension in Gegenwart von Porösmachern und Radikalbildnern in an sich bekannter Weise perlpolymerisiert und die polymerisierbaren Glycidylverbindungen vor oder während der Polymerisation mit Aminen umsetzt, die mit mindestens zwei Epoxygruppen zu reagieren vermögen.

Als Amine, die mit mindestens zwei Epoxygruppen zu reagieren vermögen seien folgende Verbindungsklassen genannt:

primäre aliphatische, cycloaliphatische, araliphatische oder aromatische Monoamine: aliphatische, cycloaliphatische, araliphatische oder aromatische Di- oder Polyamine mit mindestens zwei primären oder sekundären Aminogruppen: ferner aliphatische, cycloaliphatische, araliphatische oder aromatische Hydroxyamine mit primärer Aminogruppe.

Als Vertreter dieser Verbindungsklassen seien beispielsweise genannt:

primäre aliphatische Monoamine: $C_1$-$C_{20}$-Alkylamine, wie Ethyl-, Butyl-, i-Pentyl-, n-Hexyl-, 2-Ethylhexyl-, Dodecyl-, Palmityl- und Stearyl-amin.

Primäre cycloaliphatische Monoamine: Cyclohexylamin, Cycloheptylamin, 2,4-Dimethyl-cyclohexylamin:

primäre araliphatische Amine: Benzylamin, 2- und 4-Methyl-benzylamin;

primäre aromatische Amine: Anilin, o-, m- und p-Toluidine, Xylidine;

aliphatische Di- und Polyamine mit mindestens zwei an (einer) Aminogruppe(n) befindlichen Wasserstoffatomen: $C_2$-$C_6$-Alkylendiamine wie Ethylen-, Propylen-, Butylen-, Hexylen- und N,N-Dimethyl-ethylen-diamin, Poly-$C_2$-$C_4$-alkylen-polyamine wie Diethylentriamin, Dipropylentriamin, Bis-(3-aminopropyl)-methylamin, Tris-(3-aminopropyl)-amin, Triethylentetramin und Tripropylentetramin;

cycloaliphatische Di- und Polyamine mit mindestens zwei an (einer) Aminogruppe(n) befindlichen Wasserstoffatomen: 1,3- und 1,4-Diaminocyclohexan, 1,2-Bis-(4-aminocyclohexyl)-ethan, 1,2-Bis-(4-aminocyclohexyl)-ether, 1,2-Bis-(4-aminocyclohexyl)-methylamin und 3,3-Dimethyl-5-methyl-5-aminomethyl-cyclohexylamin;

araliphatische Di- und Polyamine mit mindestens an zwei (einer) Aminogruppe(n) befindlichen Wasserstoffatomen: 1,3- und 1,4-Bis-(aminomethyl)-benzol;

aromatische Di- und Polyamine mit mindestens zwei an (einer) Aminogruppe(n) befindlichen Wasserstoffatomen: p-Phenylendiamin, 1,2-Bis-(4-aminophenyl)-ethan, 1,2-Bis-(4-aminophenyl)-ether und 1,2-Bis-(4-aminophenyl)-methylamin, Bis-(4-aminophenyl)-methan.

Aliphatische Hydroxyamine mit primärer Aminogruppe: 2-Aminoethanol-(1), 3-Aminopropanol-(1), 4-Aminobutanol-(1) und 2-Amino-2-hydroxymethyl-propandiol-1,3;

cycloaliphatische Hydroxyamine mit primärer Aminogruppe: 4-Aminocyclohexanol-(1) und 2,2-Dimethyl-4-amino-cyclohexanol-(1);

araliphatische Hydroxyamine mit primärer Aminogruppe: 4-Amino-benzylamin.

Bevorzugt werden $C_2$-$C_6$-Alkylendiamine und Poly-$C_2$-$C_4$-alkylen-polyamine verwendet.

Die Amine werden in einer solchen Menge eingesetzt, daß nur ein Teil, etwa 5 bis 90 Mol-%, vorzugsweise 15 bis 45 Mol-% der in den polymerisierbaren Glycidyl-Verbindungen enthaltenen Epoxygruppen umgesetzt werden. Das heißt, die Amine werden in einer solchen Menge eingesetzt, daß der Gehalt der vernetzten Epoxy- und basischen Aminogruppen aufweisenden Mischpolymerisate an Epoxygruppen 1 bis 27 Gew.-%, vorzugsweise 5 bis 20 Gew.-% bezogen auf das Gewicht des Perlpolymerisates beträgt.

Zur Erhöhung der mechanischen Stabilität und eine Erzeugung der gewünschten Morphologie der Perlpolymerisate und/oder zur Einstellung eines gewissen Hydrophobitätsgrades, kann es vorteilhaft sein, die Polyglycidylverbindungen nicht nur durch Reaktionen mit den Aminen zu vernetzen, sondern zusätzlich noch mit radikalisch polymerisierbaren Polyvinylverbindungen. Als radikalisch polymerisierbare Polyvinylverbindungen kommen vor allem aromatische Divinylverbindungen wie Divinylbenzol und Divinyltoluol, aliphatische Divinylverbindungen wie Hexadien-1,5, Heptadien-1,6, Ethylenglykoldimethacrylat, Methylendi-(meth)acrylat und Ethylendivinylharnstoff, aromatische Trivinylverbindungen wie Trivinylbenzol, aliphatische Trivinylverbindungen z.B. sich von mehrwertigen Alkoholen, wie Glycerin, ableitende Acrylsäureester und Triallylcyanurat in Betracht.

Die radikalisch polymerisierbaren Polyvinylverbindungen werden in einer Menge von 0 bis 35 Gew.-%, bezogen auf das Gesamtgewicht der polymerisierbaren Monomeren eingesetzt.

Als Porösmacher werden die aus der Herstellung makroporöser Ionenaustauscher-Matrices bekannten Porösmacher verwendet. Es sind dies organische Verbindungen, die sich im Monomeren lösen, aber das Polymere ausfällen und gegebenenfalls anquellen (Fällmittel bzw. Quellmittel für das Polymere), z.B. aliphatische Kohlenwasserstoffe wie Isododecan, Octan, Decan. Dodecan, Cyclohexan; alkylsubstituierte aromatische Kohlenwasserstoffe wie Toluol; $C_4$-$C_{14}$-Alkohole wie Hexanol, Cyclohexanol, Octanol, Decanol und Methylisobutylcarbinol; aliphatische und aromatische Chlorkohlenwasserstoffe wie 1,1,1-Trichlorethan, Ethylenchlorid, Chlorbenzol und o-Dichlorbenzol oder $C_1$-$C_{18}$-Carbonsäureester wie Essigsäureethylester, Essigsäurebutylester und Stearinsäureamylester.

Die Porösmacher werden in einer Menge von 50 bis 200 Gew.-%, vorzugsweise 90 bis 150 Gew.-%, bezogen auf das Gesamtgewicht der Monomeren, eingesetzt.

Die Porösmacher werden nach der Herstellung des Perlpolymerisates durch Verdampfen oder Elution aus diesem entfernt.

Die Herstellung der erfindungsgemäßen perlförmigen, vernetzten, makroporösen Epoxy- und basische Aminogruppen aufweisenden Mischpolymerisate kann in verschiedener Weise erfolgen, je nach dem, ob man die Umsetzung der polymerisierbaren Glycidylverbindung mit dem Amin vor oder während der Perlpolymerisation vornimmt.

Gemäß Arbeitsweise (a) (Umsetzung der polymerisierbaren Glycidylverbindung mit dem Amin vor der Perlpolymerisation) werden zunächst polymerisierbare Glycidylverbindung und Amin bei Temperaturen von 0 bis 100°C, vorzugsweise 20 bis 70°C, gegebenenfalls in Gegenwart des Porösmachers oder eines anderen Verdünnungsmittels, umgesetzt. Die Lösung des Umsetzungsproduktes wird anschließend mit der wäßrigen Polymerisationsflotte und dem Gemisch aus Polyvinylverbindung, Radikalbildner und - falls die Umsetzung von Glycidylverbindung mit dem Amin in Abwesenheit des Porösmachers durchgeführt wurde - dem Porösmacher versetzt. Wird keine Polyvinylverbindung verwendet, so wird der für die Polymerisation benötigte Radikalbildner entweder im Porösmacher oder in einem Teil der polymerisierbaren Glycidylverbindung gelöst. Die erhaltenen wäßrigen Suspensionen werden zum Auslösen der Polymerisationsreaktion auf 50 bis 100°C erwärmt und so lange auf der gewählten Polymerisationstemperatur gehalten, bis die Polymerisation beendet ist. Nach dem Abkühlen wird das entstandene Perlpolymerisat mechanisch, z.B. durch Filtrieren, abgetrennt, zunächst mit Methanol dann mit Wasser gewaschen und gegebenenfalls getrocknet.

Arbeitsweise (b) (Umsetzung der polymerisierbaren Glycidylverbindung mit dem Amin während der Perlpolymerisation):

Sie ist besonders einfach durchzuführen; bei ihr werden die zu polymerisierenden Monomeren, das Amin, der Porösmacher und der Radikalbildner bei Raumtemperatur zur wäßrigen Flotte gegeben. Die wäßrige Suspension wird anschließend wie unter Arbeitsweise (a) beschrieben zum Auslösen der Polymerisation auf 50 bis 100°C erwärmt und anschließend so lange bei der gewählten Polymerisationstemperatur gerührt, bis die Polymerisation beendet ist. Die Aufarbeitung des erhaltenen Perlpolymerisats erfolgt wie unter (a) beschrieben.

Die Perlpolymerisate können Lösungsmittel-feucht oder wasserfeucht aufbewahrt werden.

Trotz der Wasserempfindlichkeit der Epoxygruppen sinkt der Epoxygruppengehalt der Harze selbst bei einer Lagerung von 6 Monaten nur um 20 %.

Die erfindungsgemäßen perlförmigen, vernetzten, makroporösen Epoxy- und basische Aminogruppen aufweisenden Mischpolymerisate eignen sich hervorragend zum Binden biologisch aktiver Substanzen wie Aktivatoren, Inhibitoren, Antigenen, Antikörpern, Vitaminen, Antibiotika, Proteinen, lebenden oder abgetöteten Zellen und insbesondere von Enzymen, z.B. Proteasen, wie Chymotrypsin oder Papain, Invertasen, Esterasen, Ligasen, Glucose-Isomerase, Lactase, Glucose-6-phosphat-dehydrogenase u.a..

Durch Hydrolyse der Epoxygruppen in den erfindungsgemäßen Mischpolymerisaten, dies kann z.B. durch Behandeln der Perlpolymerisate mit wäßrigen Lösungen, die einen pH-Wert von 1 bis 3 aufweisen, bei Temperaturen von 25 bis 100°C erreicht werden, werden Hydroxy- und basische Aminogruppen aufweisende Harze erhalten. Diese Hydroxy- und basische Aminogruppen-haltigen stark hydrophilen Perl-Mischpolymerisate eignen sich z. B. als Chromatographieharze für Biomaterialien.

Die erfindungsgemäßen perlförmigen, vernetzten, makroporösen Epoxy- und basische Aminogruppen aufweisenden Mischpolymerisate lassen sich ferner durch Umsetzung der Epoxygruppen mit Aminen und anschließendes Quaternieren der basischen Aminogruppen in den so erhaltenen schwachbasischen Anionenaustauschern in starkbasische Anionenaustauscher überführen. Diese Anionenaustauscher zeichnen sich nicht nur durch ihr ausgezeichnetes Bindungsvermögen für kolloidale Kieselsäure sondern auch durch ihre hervorragende Regenerierbarkeit aus.

Die Herstellung von Anionenaustauschern durch Umsetzung von Epoxygruppen tragenden Mischpolymerisaten mit Aminen ist zwar an sich bekannt. So wird in Angew. Makromol. Chemie 96 (1981) Seiten 69-84 die Herstellung schwachbasischer Anionenaustauscher durch Umsetzung makroporöser Mischpolymerisate aus Glycidylmethacrylat und Ethylenglykol-di-methacrylat mit Aminen und in der Angew. Makromol. Chemie 63 (1977) Seiten 23-36 die Herstellung starkbasischer Anionenaustauscher durch Umsetzung von makroporösen Mischpolymerisaten aus Glycidylmethacrylat und Ethylenglykol-di-methacrylat mit Aminen und anschließendes Quaternieren mit Ethylenchlorhydrin beschrieben. Aber weder die schwachbasischen noch die starkbasischen Anionenaustauscher sind für die Adsorption kolloidaler Kieselsäure geeignet.

Für das Binden biologisch aktiver Substanzen kann es vorteilhaft sein die erfindungsgemäßen Mischpolymerisate mit sogenannten Spacern zu versehen und anschließend die biologisch aktiven Substanzen in bekannter Weise mit Hilfe von Glutaraldehyd zu binden.

Beispiel 1

190 g (1,34 Mol) Glycidylmethacrylat und 10 g (0,17 Mol) Ethylendiamin werden 3 Stunden bei 40°C gerührt. Das Gemisch wird anschließend mit der Lösung von 2 g Azodiisobutyronitril in 200 g Butylacetat und 1000 ml 0,2 gew.-%iger wäßriger Methylcellulose-Lösung versetzt. Die Suspension wird kurze Zeit bei Raumtemperatur gerührt, dann auf 70°C erwärmt und 15 Stunden bei dieser Temperatur gerührt.

Nach beendeter Polymerisation wird das Perlpolymerisat abgesaugt und in einer Chromatographiesäule zuerst mit Methanol dann mit Wasser gründlich eluiert.

Es werden 600 ml Perlpolymerisat erhalten. Gehalt des getrockneten Perlpolymerisates an
Epoxygruppen: 15,0 Gew.-%
basischen Aminogruppen: 2,2 Gew.-%

Beispiel 2

Die Mischung aus 190 g (1,34 Mol) Glycidylmethacrylat, 10 g (0,17 Mol) Ethylendiamin, 200 g 1 %iger Lösung von Azodiisobutyronitril in Butylacetat und 1000 ml 0,2 %iger wäßriger Methylcellulose-Lösung wird kurze Zeit bei Raumtemperatur gerührt. Anschließend wird die Suspension auf 70°C erwärmt und 15 Stunden auf dieser Temperatur gehalten. Nach beendeter Perlpolymerisation und Abtrennen des Perlpolymerisates werden 450 ml Perlpolymerisat erhalten.

Gehalt des getrockneten Perlpolymerisates an
Epoxygruppen: 19,9 Gew.-% = 1,59 Mol Epoxygruppen/l Harz basischen Aminogruppen: 1,1 Gew.-%.

Beispiel 3

180 g (1,27 Mol) Glycidylmethacrylat und 20 g (0,33 Mol) Ethylendiamin werden 3 Stunden bei 40°C gerührt. Anschließend wird das Gemisch mit der Lösung von 2 g Azodiisobutyronitril in 200 g Butylacetat und 1000 ml 0,2 %iger wäßriger Methylcellulose-Lösung versetzt. Die Suspension wird kurze Zeit bei Raumtemperatur gerührt, dann auf 70°C erwärmt und 15 Stunden auf dieser Temperatur gehalten.

Das Perlpolymerisat wird wie in Beispiel 1 beschrieben isoliert. Es werden 595 ml Perlpolymerisat erhalten. Gehalt des Polymerisates an
Epoxygruppen: 7,7 Gew.-%
basischen Aminogruppen: 4,4 Gew.-%

Beispiel 4

180 g (1,27 Mol) Glycidylmethacrylat, 10 g (0,17 Mol) Ethylendiamin werden 3 Stunden bei 40°C gerührt. Anschließend wird das Gemisch mit der Lösung von 2 g Azodiisobutyronitril in 200 g Butylacetat, 10 g (0,5 Mol) Ethylenglykoldimethacrylat und 1000 ml 0,2 %iger wäßriger Methylcellulose-Lösung versetzt. Die Suspension wird kurze Zeit bei Raumtemperatur gerührt dann auf 70°C erwärmt und 15 Stunden auf dieser Temperatur gehalten. Anschließend wird das Perlpolymerisat wie in Beispiel 1 beschrieben isoliert; es werden 610 ml Perlpolymerisat erhalten. Gehalt des Perlpolymerisates an
Epoxygruppen: 14,2 Gew.-%
basischen Aminogruppen: 1,9 Gew.-%

Beispiel 5

180 g (1,27 Mol) Glycidylmethacrylat, 10 g (0,17 Mol) Ethylendiamin, 10 g (0,07 Mol) Divinylethylenharnstoff und die Lösung von 2 g Azodiisobutyronitril werden in 1000 ml 0,2 %iger wäßriger Methylcellulose-Lösung bei Raumtemperatur dispergiert. Die Suspension wird auf 70°C erwärmt und 15 Stunden auf dieser Temperatur gehalten.

Anschließend wird das Perlpolymerisat wie in Beispiel 1 beschrieben isoliert; es werden 520 ml Perlpolymerisat erhalten. Gehalt des Perlpolymerisates an
Epoxygruppen: 18,5 Gew.-%
basischen Aminogruppen: 1 Gew.-%

Beispiel 6

Die Lösung von 127 g (0,89 Mol) Glycidylmethacrylat und 10 g (0,17 Mol) Ethylendiamin in 300 g Methylisobutylcarbinol wird 3 Stunden bei 50°C gerührt. Anschließend wird die Lösung mit 63 g (0,30 Mol) Divinylbenzol (62 %ig), 2 g Azodiisobutyronitril und 1500 ml 0,2 %iger wäßriger Methylcellulose-Lösung versetzt. Die Suspension wird kurze Zeit bei Raumtemperatur gerührt, dann auf 70°C erwärmt und 15 Stunden auf dieser Temperatur gehalten. Das Perlpolymerisat wird wie in Beispiel 1 beschrieben isoliert: es werden 950 ml Perlpolymerisat erhalten. Gehalt des Perlpolymerisates an
Epoxygruppen: 7,1 Gew.-%
basischen Aminogruppen: 0,9 Gew.-%

Beispiel 7

190 g (1,34 Mol) Glycidylmethacrylat und 10 g (0,16 Mol) Ethanolamin werden 3 Stunden bei 40°C gerührt. Anschließend wird die Lösung von 2 g Azodiisobutyronitril in 200 g Butylacetat und 1000 ml 0,2 %iger wäßriger Methylcellulose-Lösung zugegeben. Die Suspension wird kurze Zeit bei Raumtemperatur gerührt, dann auf 70°C erwärmt und 15 Stunden auf dieser Temperatur gehalten. Das Perlpolymerisat wird wie in Beispiel 1 beschrieben isoliert. Es werden 840 ml Perlpolymerisat erhalten.
Gehalt des Perlpolymerisates an
Epoxygruppen: 11,9 Gew.-%
basischen Aminogruppen: 1,0 Gew.-%

Beispiel 8

190 g (1,34 Mol) Glycidylmethacrylat, 20 g (0,22 Mol) 50 %ige wäßrige Ethylaminlösung, und die Lösung von 2 g Azodiisobutyronitril in 200 g Butylacetat werden zusammen mit 1000 ml 0,2 %iger wäßriger Methylcellulose-Lösung verrührt. Die Suspension wird auf 70°C erhitzt und 15 Stunden auf dieser Temperatur gehalten. Das Perlpolymerisat wird wie in Beispiel 1 beschrieben isoliert. Es werden 540 ml Perlpolymerisat erhalten.
Gehalt des Perlpolymerisates an
Epoxygruppen: 13,9 Gew.-%
basischen Aminogruppen: 0,6 Gew.-%

Beispiel 9

250 g gemäß Beispiel 1 hergestelltes und anschließend getrocknetes Perlpolymerisat werden mit 1500 g 3-Dimethylaminopropylamin 20 Stunden bei 40°C gerührt. Das Harz wird durch Absaugen vom überschüssigen Amin abgetrennt und anschließend zunächst mit Methanol dann mit Wasser neutral gewaschen. Es werden 1220 ml schwach basischer Anionenaustauscher (Totalkapazität $T_K$ 1.52 val/l Harz) erhalten.
400 ml dieses schwach basischen Anionenaustauschers werden mit 266 g 45 %iger NaOH, 800 ml entioniosiertem Wasser und 108,5 ml Methylchlorid 16 Stunden bei 40°C im Autoklaven gerührt. Es werden 1100 ml eines stark basischen Anionenaustauschers ($T_K$: 0,4 val/l Harz) erhalten.
Der mittlere Porenradius des stark basischen Anionenaustauschers beträgt 2,0 $\mu$.

Beispiel 10

100 ml des gemäß Beispiel 8 hergestellten Harzes werden in verdünnter Schwefelsäure 20 h bei 100°C gerührt und anschließend mit vollentsalztem Wasser neutral gewaschen. Das so modifizierte Harz wird anschließend für folgende chromatographischen Trennungen verwendet:

a) **Entsalzung**

**Trennbedingungen:**

Säule: Länge:  46.4 cm

Innendurchmesser:  1.5 cm

Teilchendurchmesser:  150-500μ

Aufgabevolumen: 0.25 ml 4 % Albumin, 2 % NaCl

Elution: Phosphatpuffer, pH 8

Durchflußgeschwindigkeit: 1,5 ml/min.

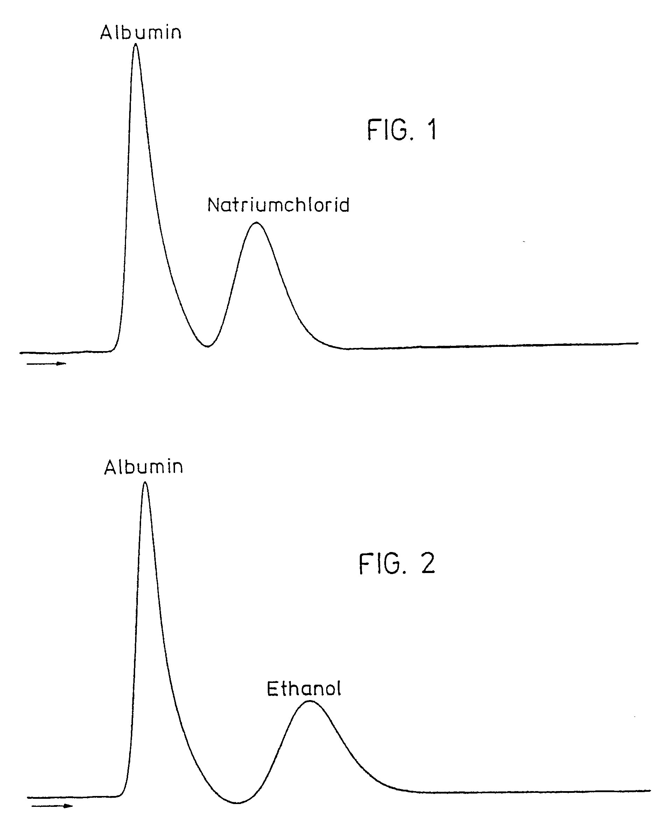

Das Ergebnis der Trennung ist in Fig. 1 dargestellt.

b) **Abtrennung organischer Lösungsmittel**

**Trennbedingungen:**

Säule: Länge:  46.4 cm

Innendurchmesser:  1.5 cm

Teilchendurchmesser:  150-200μ

Aufgabevolumen: 0.25 ml 4 % Albumin, 2 % Ethanol

Elution: Phosphatpuffer, pH 8

Durchflußgeschwindigkeit: 1,5 ml/min.

Das Ergebnis der Trennung ist in Fig. 2 dargestellt.

Beispiel 11

1 g des gemäß Beispiel 3 hergestellten Harzes wird mit 50 ml einer Esterase-Lösung (633 U) in 0.1 molarem Phosphatpuffer (pH 8.5) 36 h bei Raumtemperatur geschüttelt. Anschließend wird das Harz abgesaugt und mit Puffer und Wasser gründlich gewaschen. Die Kupplungsausbeute an Esterase beträgt 31.2 %.

Wird anstelle des verwendeten Harzes das in Beispiel 1 der EP-B1-0 058 767 beschriebene Harz verwendet, so beträgt die Kupplungsausbeute an Esterase nur 17 %.

**Ansprüche**

1. Perlförmige vernetzte makroporöse Epoxy- und basische Aminogruppen aufweisende Mischpolymerisate dadurch erhältlich, daß man polymerisierbare Glycidylverbindungen vor oder während der radikalischen Perlpolymerisation, die in Gegenwart von Porösmachern und gegebenenfalls unter Zusatz von radikalisch polymerisierbaren Polyvinylverbindungen vorgenommen wird, mit Aminen umsetzt, die mit mindestens zwei Epoxygruppen zu reagieren vermögen.

2. Perlförmige Mischpolymerisate gemäß Anspruch 1, dadurch gekennzeichnet, daß man als polymerisierbare Glycidylverbindungen Glycidyl-(meth)acrylate, Glycidylvinyl- und/oder Glycidyl-allylether verwendet.

3. Perlförmige Mischpolymerisate gemäß Anspruch 1 und 2, dadurch gekennzeichnet, daß als Amine, die mit mindestens zwei Epoxygruppen zu reagieren vermögen, primäre aliphatische, cycloaliphatische, araliphatische oder aromatische Monoamine, aliphatische, cycloaliphatische, araliphatische oder aromatische Di- oder Polyamine mit mindestens zwei an (einer) Aminogruppe(n) befindlichen Wasserstoffato-

7

men oder aliphatische, cycloaliphatische, araliphatische oder aromatische Hydroxyamine mit primärer Aminogruppe verwendet werden.

4. Perlförmige Mischpolymerisate gemäß Anspruch 1 und 2, dadurch gekennzeichnet, daß als Amine $C_2$-$C_6$-Alkylendiamine und Poly-$C_2$-$C_4$-alkylen-polyamine verwendet werden.

5. Perlförmige Mischpolymerisate gemäß Anspruch 1 bis 4, dadurch gekennzeichnet, daß die Amine in einer Menge eingesetzt werden, daß der Gehalt der vernetzten Epoxy- und basische Aminogruppen aufweisenden Mischpolymerisate an Epoxygruppen 1 bis 27 Gew.-% beträgt.

6. Perlförmige Mischpolymerisate gemäß Anspruch 1 bis 5, dadurch gekennzeichnet, daß man den Porösmacher in einer Menge von 50 bis 200 Gew.-%, bezogen auf das Gesamtgewicht der Monomeren einsetzt.

7. Perlförmige Mischpolymerisate gemäß Ansprüchen 1 bis 6, dadurch gekennzeichnet, daß man als radikalisch polymerisierbare Polyvinylverbindungen aliphatische und/oder aromatische Di- und Trivinylverbindungen und/oder Triallylcyanurat verwendet.

8. Perlförmige Mischpolymerisate gemäß Ansprüchen 1 bis 7, dadurch gekennzeichnet, daß man die radikalisch polymerisierbaren Polyvinylverbindungen in einer Menge von 0 bis 35 Gew.-%, bezogen auf das Gesamtgewicht an polymerisierbaren Monomeren einsetzt.

9. Verfahren zur Herstellung von perlförmigen, vernetzten, makroporösen Epoxy- und basische Aminogruppen aufweisenden Mischpolymerisaten, dadurch gekennzeichnet, daß man polymerisierbare Glycidylverbindungen, gegebenenfalls unter Zusatz von radikalisch polymerisierbaren Polyvinylverbindungen, in wäßriger Suspension in Gegenwart von Porösmachern und Radikalbildnern in an sich bekannter Weise perlpolymerisiert und die polymerisierbaren Glycidylverbindungen vor oder während der Polymerisation mit Aminen umsetzt, die mit mindestens zwei Epoxygruppen zu reagieren vermögen.

10. Verwendung der perlförmigen, vernetzten, makroporösen Epoxy- und basische Aminogruppen aufweisenden Mischpolymerisate zum Binden von biologisch aktiven Substanzen, zur Herstellung stark basischer Anionenaustauscher und hydrophiler Chromatographie-Harze für Biomaterialien.

## Claims

1. Bead-shaped, crosslinked, macroporous copolymers containing epoxide groups and basic amino groups, obtainable by reacting polymerisable glycidyl compounds, before or during free radical bead polymerisation, which is carried out in the presence of agents which impart porosity and, if appropriate, with the addition of polyvinyl compounds which can be polymerised by free radical polymerisation, with amines which are capable of reacting with at least two epoxide groups.

2. Bead-shaped copolymers according to Claim 1, characterised in that glycidyl (meth)acrylates or glycidyl vinyl and/or glycidyl allyl ethers are used as the polymerisable glycidyl compounds.

3. Bead-shaped copolymers according to Claims 1 and 2, characterized in that primary aliphatic, cycloaliphatic, araliphatic or aromatic monoamines, aliphatic, cycloaliphatic, araliphatic or aromatic di- or polyamines with at least two hydrogen atoms on (one) amino group(s) or aliphatic, cycloaliphatic, araliphatic or aromatic hydroxylamines with a primary amino group are used as the amines which are capable of reacting with at least two epoxide groups.

4. Bead-shaped copolymers according to Claims 1 and 2, characterized in that $C_2$-$C_6$-alkylenediamines and poly-$C_2$-$C_4$-alkylene-polyamines are used as the amines.

5. Bead-shaped copolymers according to Claims 1 to 4, characterized in that the amines are employed in an amount such that the content of epoxide groups in the crosslinked copolymers containing epoxide groups and basic amino groups is 1 to 27% by weight.

8

6. Bead-shaped copolymers according to Claims 1 to 5, characterized in that the agent which imparts porosity is employed in an amount of 50 to 200% by weight, based on the total weight of the monomers.

7. Bead-shaped copolymers according to Claims 1 to 6, characterized in that aliphatic and/or aromatic diand trivinyl compounds and/or triallyl cyanurate are used as the polyvinyl compounds which can be polymerised by free radical polymerisation.

8. Bead-shaped copolymers according to Claims 1 to 7, characterized in that the polyvinyl compounds which can be polymerised by free radical polymerisation are employed in an amount of 0 to 35% by weight, based on the total weight of polymerisable monomers.

9. Process for the preparation of bead-shaped, crosslinked, macroporous copolymers containing epoxide groups and basic amino groups, characterized in that polymerisable glycidyl compounds are subjected to bead polymerisation in a manner which is known per se, if appropriate with the addition of polyvinyl compounds which can be polymerised by free radical polymerisation, in an aqueous suspension in the presence of agents which impart porosity and agents which form free radicals, and the polymerisable glycidyl compounds are reacted, before or during the polymerisation, with amines which are capable of reacting with at least two epoxide groups.

10. Use of the bead-shaped, crosslinked, macroporous copolymers containing epoxide groups and basic amino groups for binding biologically active substances, for the preparation of strongly basic anion exchangers and hydrophilic chromatography resins for biomaterials.

**Revendications**

1. Copolymérisats porteurs de groupes époxy et amino basiques, macroporeux, réticulés, en forme de perles, obtenus par le fait qu'on fait réagir des composés de glycidyle polymérisables avant ou pendant la polymérisation radicalaire en suspension, que l'on conduit en présence d'agents porogènes et, le cas échéant, avec addition de composés polyvinyliques susceptibles de polymérisation radicalaire, avec des amines qui peuvent réagir avec au moins deux groupes époxy.

2. Copolymérisats en perles suivant la revendication 1, caractérisés en ce qu'on utilise comme composés de glycidyle polymérisables des (méth)acrylates de glycidyle, des éthers de glycidyle et de vinyle et/ou des éthers de glycidyle et d'allyle.

3. Copolymérisats en perles suivant les revendications 1 et 2, caractérisés en ce qu'on utilise comme amines qui peuvent réagir avec au moins deux groupes époxy, des mono-amines primaires aliphatiques, cycloaliphatiques, araliphatiques ou aromatiques, des diamines ou des polyamines aliphatiques, cycloaliphatiques, araliphatiques ou aromatiques portant au moins deux atomes d'hydrogène se trouvant sur un ou des groupes amino ou des hydroxyamines aliphatiques, cycloaliphatiques, araliphatiques ou aromatiques portant un groupe amino primaire.

4. Copolymérisats en perles suivant les revendications 1 et 2, caractérisés en ce qu'on utilise comme amines des (alkylène en $C_2$ à $C_6$)-diamines et des poly-(alkylène en $C_2$ à $C_4$)-polyamines.

5. Copolymérisats en perles suivant les revendications 1 à 4, caractérisés en ce qu'on utilise les amines en une quantité telle que la teneur en groupes époxy des copolymérisats réticulés porteurs de groupes époxy et de groupes amino basiques soit de 1 à 27 % en poids.

6. Copolymérisats en perles suivant les revendications 1 à 5, caractérisés en ce qu'on utilise l'agent porogène en une quantité de 50 à 200 % en poids par rapport au poids total des monomères.

7. Copolymérisats en perles suivant les revendications 1 à 6, caractérisés en ce qu'on utilise comme composés polyvinyliques susceptibles de polymérisation radicalaire des composés de divinyle et de trivinyle aliphatiques et/ou aromatiques et/ou le cyanurate de triallyle.

8. Copolymérisats en perles suivant les revendications 1 à 7, caractérisés en ce qu'on utilise les composés polyvinyliques susceptibles de polymérisation radicalaire en une quantité de 0 à 35 % en poids par rapport au poids total de monomères polymérisables.

9. Procédé de production de copolymérisats en perles réticulés, macroporeux, porteurs de groupes époxy et de groupes amino basiques, caractérisé en ce qu' on polymérise en suspension des composés glycidyliques polymérisables, éventuellement avec addition de composés polyvinyliques susceptibles de polymérisation radicalaire, en suspension aqueuse en présence d'agents porogènes et de générateurs de radicaux, d'une manière connue, et on fait réagir les composés de glycidyle polymérisables avant ou pendant la polymérisation avec des amines qui peuvent réagir avec au moins deux groupes époxy.

10. Utilisation des copolymérisats en perles réticulés, macroporeux, porteurs de groupes époxy et amino basiques pour la liaison de substances douées d'activité biologique, en vue de la préparation d'échangeurs anioniques fortement basiques et de résines chromatographiques hydrophiles pour des matières biologiques.

Albumin

Natriumchlorid

FIG. 1

Albumin

Ethanol

FIG. 2